# EUROPEAN PATENT SPECIFICATION

(11) **EP 1 075 850 B1**
(45) Date of publication and mention of the grant of the patent: **01.09.2004**
(21) Application number: 00120492.4
(22) Date of filing: 17.04.1995
(51) Int. Cl.: A61M 25/06, A61M 5/32

(54) **Apparatus for intravenous catheter insertion**
Vorrichtung zum intravenösen Einführen eines Katheters
Dispositif pour l'insertion intraveineuse d'un cathéter

(43) Date of publication of application: 14.02.2001
(62) Divisional of application: 95916338.7
(73) Proprietor: MDC Investment Holding, Inc., Wilmington, DE 19801 (US)
(72) Inventor: Botich, Michael J., Oxnard, CA 93065 (US); Halseth, Thor R., Simi Valley, CA 93065 (US)
(74) Representative: Newby, Martin John

(56) References cited:
- EP-A- 0 645 159
- WO-A-93/12830
- US-A- 5 295 975
- US-A- 5 376 075

## Description

The present invention relates to an apparatus for intravenous catheter insertion having a retractable needle for rendering the needle safely out of exposure immediately after use of the device. The invention is useful in the practice of medicine for improving safety from the danger of needle pricks after the intended use of the apparatus in inserting an intravenous catheter.

Intravenous catheters are employed in the practice of medicine for intravenous administration of fluids, such as hydrating solutions, medications, blood products, and nutrients, to a patient. An intravenous catheter ordinarily includes a flexible tube, or cannula, having a tip that is positioned within a blood vessel of the patient. The cannula extends from the tip to a location external to the body of the patient. A connection hub is attached to the external end of the cannula for connection to external apparatus to facilitate intravenous therapy. Usually, the connection hub is attached to tubing for delivery of parenteral fluids from a source of fluid. The tubing may have an injection port with an anticoagulant reservoir (e.g. a heparin lock) for periodic administration of medication, for example.

Prior to insertion into a patient, an intravenous catheter is provided as a part of a trocar. The trocar further includes a stylet having a needle. The needle extends from the forward end of the stylet and through the connection hub and catheter cannula, which is supported as a sleeve over the needle. The connection hub may be removably engaged with the forward end of the stylet. The tip of the needle when the cannula is positioned thereon, extends beyond the insertion end of the cannula.

In positioning the tip of the catheter within a blood vessel of the patient, a doctor or other medical personnel grasps the body of the stylet with one hand and pierces the skin of the patient with the needle to locate the tip of the cannula within a desired blood vessel. It should be apparent that the skin of the patient is typically pierced at a low angle to the plane of the skin. In use of the catheter insertion device, the device is usually gripped for insertion in an overhand manner, wherein the forward end of the stylet is held between the thumb and a finger of the doctor's dominant hand, such that the rear of the stylet extends toward the palm. The needle, bearing the catheter cannula, is then guided through the skin of the patient and into the desired blood vessel.

Correct positioning of the tip of the needle within the desired blood vessel is usually indicated by a "blood return", in which the blood pressure within the blood vessel forces a small amount of blood to flow through a cylindrical capillary between the needle and the cannula of the catheter. After a blood return is observed, the stylet is withdrawn while the doctor uses the other hand to apply light pressure upon the catheter, so that the needle can be withdrawn from within the cannula while the cannula remains in place within the blood vessel.

After the stylet is withdrawn, the doctor normally tapes the catheter in position at the insertion site prior to attaching an intravenous tube or hydraulic lock to the connection hub. Of course, in order to tape the catheter and to connect the desired apparatus to the connection hub, the doctor must put down the stylet to gain a free hand. Thus, the contaminated needle of the stylet remains an exposed sharp hazard until the doctor has finished attending to the patient and can then properly dispose of the stylet. Other health care workers in the vicinity may not be aware of the exposed contaminated needle, and could be accidentally pricked, if the needle is contacted. Additionally, the doctor may be accidentally pricked with the needle during disposal thereof. In the past, such accidental needle sticks were considered to be a routine occupational inconvenience. Now, such occurrences are recognized as a vector for lethal illnesses, including hepatitis-B and the Human Immune Virus (HIV), which is associated with Acquired Immune Deficiency Syndrome (AIDS).

Known catheter insertion devices are disclosed in US-A-5376075 and US-A-4747831. In US-A-5376075, the rear handle of the apparatus is pulled back to cause release of a needle. In US-A-4747831, a depressible plunger causes needle release but the plunger is not locked in its outer position during catheter insertion.

It would be desirable to provide an apparatus or stylet for catheter insertion that could be rendered safe immediately after withdrawal from a patient. Previous attempts to provide a catheter insertion apparatus which renders the needle safe from accidental pricks after use, have included stylets with slidable locking sleeves thereon for surrounding the needle after use. Because such a safety feature is most desirably activated when the doctor has only one free hand, activation of such sliding or telescoping sleeves have been difficult. Hence, it would be desirable to provide a catheter stylet that could quickly prevent the needle from being a hazard by use of a simple, natural movement of one hand, and requiring minimal dexterity.

Furthermore, it would be desirable to provide a catheter insertion apparatus that provides a non-distracting, yet immediate and readily discernible tactile or audible confirmation that the needle is encased so as not to present a hazard that can prick a health care worker or one responsible for disposal of used needles.

According to the present invention there is provided an apparatus for intravenous catheter insertion, the apparatus being as claimed in the ensuing claim 1.

Conveniently the release of the needle retaining member is effected by pressing the plunger against the palm of the hand while maintaining the same grip upon the device as used during catheter insertion.

Other new and useful features of the invention will be apparent from the description set forth hereinafter.

The foregoing summary, as well as the following detailed description, will be best understood when read in conjunction with the attached drawings in which:
FIG. 1 is a sectional view of a catheter insertion apparatus of the present invention;
FIG. 2A is an enlarged fragmentary sectional view of the front end of the catheter insertion apparatus of FIG. 1 with the catheter removed;
FIG. 2B is an enlarged fragmentary sectional view of the rear end of the catheter insertion apparatus of FIG. 1;
FIG. 3 is an exploded perspective view of a needle retaining mechanism for holding a spring-loaded needle within the catheter insertion apparatus of FIG. 1; and
FIG. 4 is a perspective view of an alternative embodiment of the needle retaining member for use in the catheter insertion apparatus of FIG. 1.

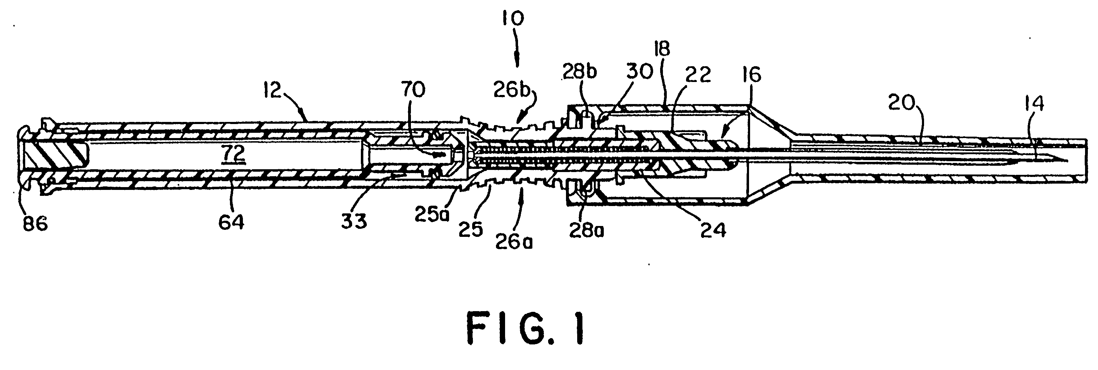

Referring now to FIG. 1, there is shown the apparatus or device of the present invention generally designated 10. The device 10 may be referred to as a trocar or stylet. The device includes a hollow housing of varying cross section 12 with a needle 14 extending therefrom with a catheter 16 and a protective hollow cap 18 encircling the needle. The needle 14 extends outwardly from the front end of the housing. The catheter 16 includes a tapered flexible cannula 20 positioned as a sleeve over the needle 14. The tip or front end of the needle 14 extends beyond the front end of the cannula. The tip of the needle 14 is preferably tapered to prevent coring of tissue when the needle is inserted into a patient. The catheter 16 further includes a connection hub 22 attached to the distal or rear end of the cannula 20. The front end of the housing 12 includes a front alignment member 24 that is contoured to mate with the connection hub 22 and removably hold the catheter 16 and its associated hub 22 in frictional engagement therewith before the catheter is inserted for use in the patient.

Finger ridges, generally designated 26a and 26b, are formed on opposite sides of the forward end 25 of the housing 12. The ridges 26a and 26b are contoured to allow a doctor or other health care professional to comfortably grip the device 10 near the forward end thereof, and preferably between the thumb and forefinger of the doctor's preferred hand for use of such devices. Ridges 28a and 28b are formed upon the forward end 25 of the housing, in front of the finger ridges 26a and 26b, for retaining the cap 18 upon the device, preferably in snap-fit engagement with retaining groove 30 formed on the interior surface of the hollow cap 18. The cap 18 extends forward from the front end 25 of the housing to surround and protect the needle 14 prior to use of the device 10.

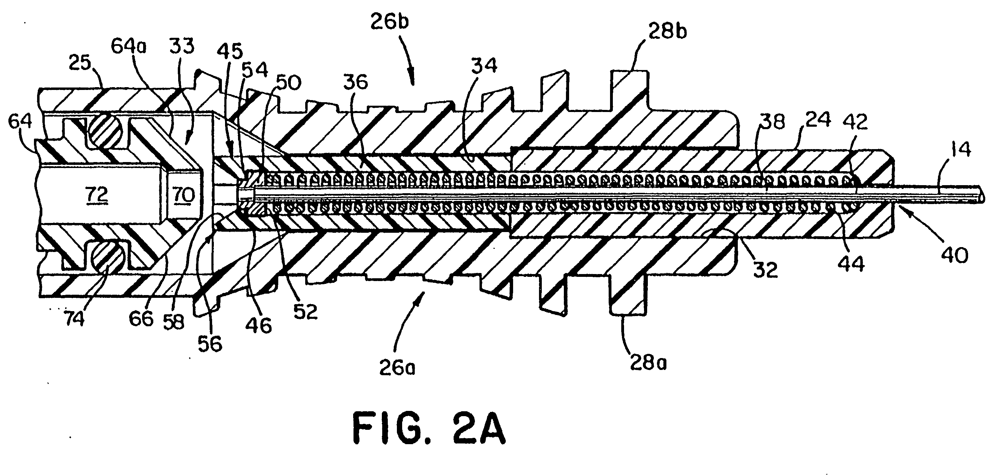

Referring now to FIG. 2A, the front or forward end of the housing 12 is shown in greater detail. The front end 25 of the housing has an axial bore 32 formed therein. The axial bore 32 is sized to receive the front alignment member 24. The front alignment member 24 is firmly engaged within the forward portion of the axial bore 32 by a friction fit. The positioning of the alignment member within the front portion of the axial bore 32 may be further secured by epoxy or ultrasonic welding. The alignment member 24 is generally cylindrical and has a cylindrical axial cavity 38 with reduced diameter portion 40 providing an opening adapted to accommodate the needle 14. The reduced diameter portion 40 also provides an internal annular surface 42, which functions as an abutment for the forward end of a compression spring 44.

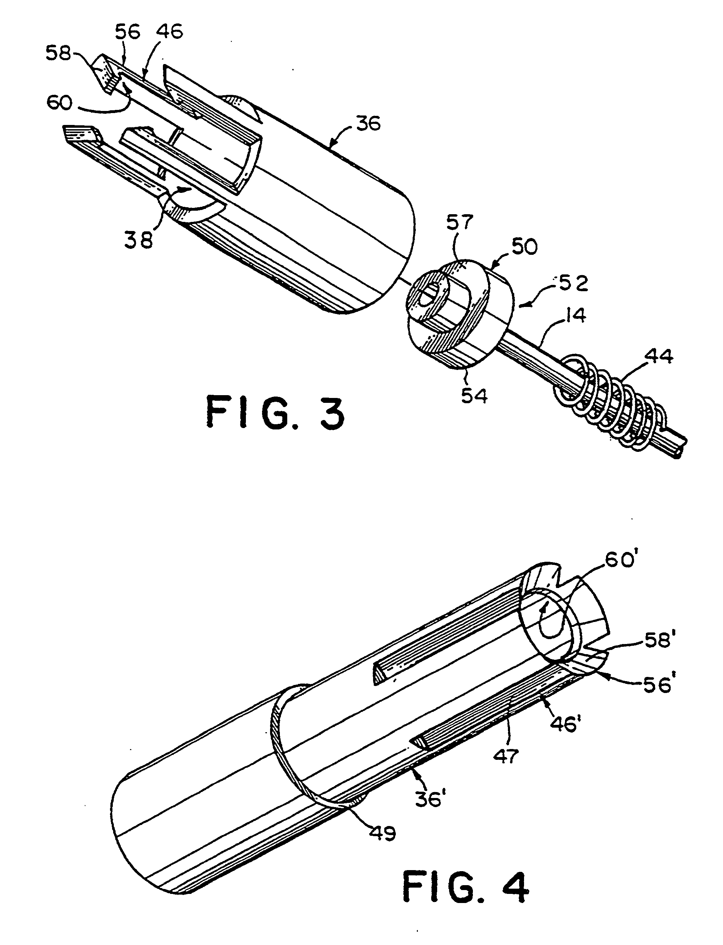

The rear or rearward end of the front alignment member 24 abuts against a reduced diameter portion 34 of the axial opening through the forward end 25 of the housing 12. A needle retaining member 36 is firmly held in the reduced diameter portion 34 of bore 32, in the forward end 25 of the housing 12. The forward end of the needle retaining member 36 abuts against the rearward end of the front alignment member 24. The rear end of the needle retaining member 36 is provided with latch means generally designated 45. The latch means is preferably provided by a plurality of latching projections or crooked fingers designated 46 formed at the rear end of the needle retaining member. The fingers 46 extend from the rear of the needle retaining member 36 into an outwardly tapered portion of the interior of the housing 12 at a junction 25a between the enlarged diameter barrel portion 33 and the reduced diameter front portion of the housing 12. The barrel extends rearwardly for the remainder of the length of the housing 12. The latching projections or crooked fingers 46, more specifically have hooks 56 integrally formed at their ends, which extend radially inward for retaining the needle 14 in position, as further described hereinbelow. In the present preferred embodiment four fingers are employed, as shown in FIG. 3, but more or less latching projections may be employed depending on the size of the device, the nature of the spring 44 and other obvious variables.

The interior of needle retaining member 36 is hollow to accommodate the needle 14 and its surrounding spring 44. It should be apparent that the axial cavity or hollow area in the needle retaining member is coextensive with the axial cavity or opening in the front alignment member 24 to accommodate the needle and its associated spring 44. The structure of the needle includes the needle 14 and an increased diameter head 50 attached thereto. The head 50 of the needle functions as a cooperating latch member with the latching projections or fingers 46. The needle head 50 includes an enlarged portion having an annular forward surface 52 which provides an abutment 55 for the rear end of spring 44 for compressing the spring with abutment 42 on the front alignment member 24. The needle head 50 includes another abutment surface 57, which is formed as a lip or rim that is maintained in abutment with the hooks 56 or fingers 46. Hence, the spring 44 is maintained in compression between the forward surface 52 of the needle head 50 and the rearward interior surface 42 of the front alignment member 24, to bias the needle toward the rear of the device.

The cooperative relationships among the needle retaining member 36, needle head 50, needle 14, and the spring 44, are best shown in the exploded view of FIG. 3. As previously discussed, the needle retaining member 36 includes rearward extending fingers or latching projections 46 having hooks 56 at the terminal ends thereof. The fingers 46 are preferably flexible to permit their outward movement to have the latching projections release the cooperating latch abutment 57 on the head 50 of the needle. It should also be apparent that the fingers could be fractured when moved outwardly to release the needle head. The hooks 56 provide engaging surfaces 60 which extend radially into the cavity 38 for engagement with the abutment surface 57 of the needle head 50.

As should be appreciated, when the fingers 46 are deformed or flexed radially outward, the engaging surfaces 60 of hooks 56 would be moved out of abutment with the abutment surface 57 of the needle head 50. Upon this occurrence, The compressive force of spring 44 against the forward surface 53 of the needle head 50, would immediately thrust the needle head 50, and hence the needle 14, rearward toward the rear of the device. Referring again to FIG. 2A, an actuating member 64 is slidably positioned within the barrel 33 of the housing 12 for effecting such disengagement of the latching structure to free the needle head 50 for having the needle fully retracted into the device.

The forward end 64a of the actuating member 64 is contoured or wedge shaped to mate with cooperating wedge shaped surfaces 58 of the hooks 56, for spreading the fingers 46 to release the latching structure. More specifically, the actuating member 64 preferably has a tapered forward end 66 which engages complementary sloping faces 58 of the hooks 56, when the actuating member is urged forward within the barrel 33 of the housing 12. The forward motion of the actuating member 64 causes the fingers 46 to spread radially outward by flexing or breaking, thus releasing the head of the needle. When the head of the needle is released, the needle is thrust rearward by the spring and is propelled through an aperture 70 in the forward end of the actuating member 64.

An O-ring 74 is held in an annular recess around the actuating member to be in sliding engagement between the actuating member and the interior of the barrel 33. The O-ring arrangement maintains the aperture 70 in alignment with the needle head for unhindered retraction of the needle. Alternatively, the actuating member may be formed to fit within the barrel and to maintain alignment therein by an integral sliding seal.

An alternative embodiment of the needle retaining member 36 is shown in FIG. 4, wherein members similar to those in FIG. 3 are shown with the same number designator with the addition of primes thereto. The latching projections or fingers 46' and the hooks 56' of the needle retaining member 36' are effectively joined together to form an annular latching member with a circular opening at the top or rear end. The retaining member is provided with V-shaped longitudinal grooves 47 running along the outside toward the rear end to facilitate breakage of the latching surface 58 by the activating member. The engaging surfaces 60' of the hook surface 56' forms a continuous rim within the interior of the needle retaining member 36', to enhance the security of engagement with the needle head. The continuous rim provides a seal with the rearward rim of the needle head, so that fluid is kept out of the needle compartment. Additionally, a radially-protruding shoulder 49 is formed around the exterior of the needle retaining member 36' for abutment with a complementary ridge on the interior of the stylet housing (not shown) to secure the needle retaining member against being pushed rearward by the expansive force of the compressed spring. When the tapered end of the actuating member 64 is urged against the sloping face 58' of hook surface 56' with sufficient force, the resulting outward radial force on the hook surface 56' serves to break the latching end of the retaining member along the grooves 47 to snap the retaining or latching end of the retaining member. More specifically, continued pressure on the actuating member forces the latching member to deform radially outward, by flexing outwardly into segments which are separated along the grooves 47 or by breaking the separated segments of the latching end to release the needle head.

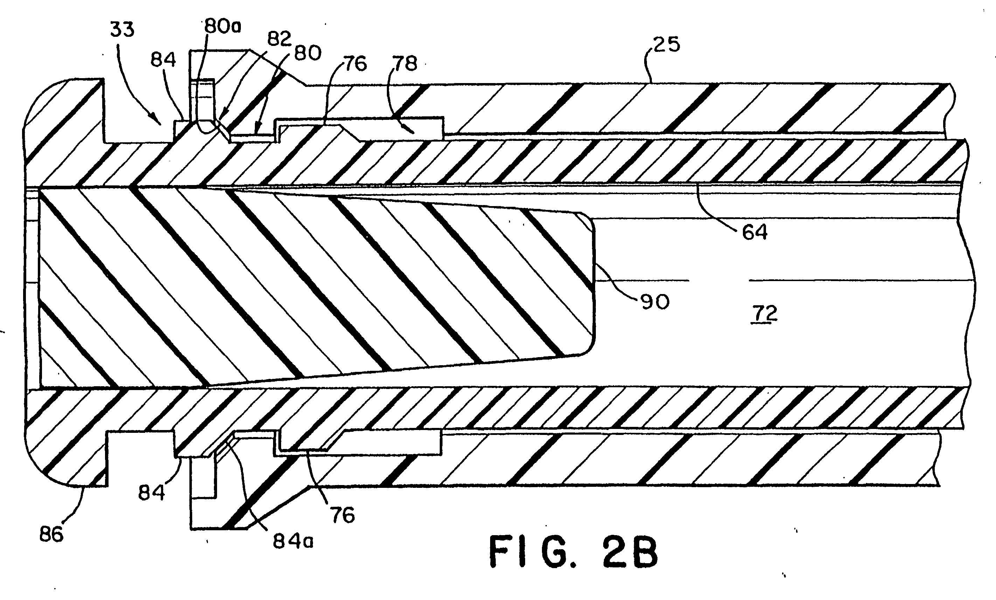

Prior to, and during insertion of the stylet and catheter into the patient, the actuating member 64 is maintained at a fixed position, so that the needle is not prematurely retracted. The actuating member 64 is maintained at a first or rearward position within the barrel, so that the rear of the actuating member protrudes from the rear of the barrel 33, as shown in FIGS. 1 and 2B. Additionally, it is preferable for the actuating member to remain locked within the barrel 33, at its second or forward position, after the needle is retracted into the device, in order to prevent access to a contaminated or used needle. Both of these objectives are attained by the dual-position locking mechanism provided at the rear end of the device, as shown most clearly in the enlarged view in FIG. 2B.

The rear of the housing 12 has an open end to receive the actuating member 64 within the barrel 33 during assembly of the device. The actuating member 64 has first locking tabs 76 thereon, which extend outward from the exterior of the actuating member 64, as in the form of a rim or tooth. The first locking tabs 76 extend slightly beyond the internal diameter of the barrel and have sloping forward surfaces thereon, to allow the tabs 76 to be forced or press-fitted into an internal circumferential groove 78 formed in the interior surface of the barrel. A lip 80 is formed in the interior of the barrel toward the open end between groove 78 and the open end of the barrel 33. Second locking tabs 84 are formed on the exterior of the actuating member 64 around its circumference, the second locking tabs 84 being located to the rear of the first locking tabs 76. When the actuating member is positioned within the barrel 33 during assembly of the stylet, the lip 80 is caught between the rear surfaces of the first locking tabs 76 and the forward surfaces of second locking tabs 84. Hence, the actuating member is thereby held at a first fixed position within the barrel 33 for initial use of the device in insertion of the catheter.

The forward surfaces 84a of the second locking tabs 84 are angled or ramped to mate with complementary angled rearward surfaces 80a of the lip 80. In activation of needle retraction, the actuating member is pushed or urged forward within the barrel 33 with sufficient force to cause the second locking tabs 84 to enter the barrel 33 by virtue of a radial deforming force exerted mutually between the angled surfaces of the second locking tabs 84 and the lip 80 Continued forward motion of the actuating member within the barrel is eventually halted by abutment of the rear end of the housing 12 with an enlarged annular stop 86 forming the rear head of the actuating member 64. In other words, when the actuating member 64 is urged forward into the barrel 33, the second locking tabs snap into the groove 78, thus producing a distinct audible and tactile sensation indicating that needle retraction has been effected. Hence, the doctor does not need to look at the stylet 12 in order to ascertain whether the needle has been retracted.

The force required to effect retraction is sufficiently high to minimize undesirable premature retraction, yet sufficiently low that the average person can effect retraction with one hand. Referring again to FIG. 1, retraction of the needle 14 is preferably effected by pressing the enlarged head or stop 86 of the actuating member 64 against the palm of the hand. As previously mentioned, the forward end of the stylet is gripped during use, between the thumb and a forefinger of the dominant hand, with the rear of the stylet aligned with the palm. In order to effect retraction, the doctor merely flexes the gripping thumb and finger firmly toward the palm while maintaining a natural grip on the stylet. Hence, the doctor does not need to be distracted from attending to the inserted catheter in order to render the stylet in a safe condition with the needle retracted and to receive confirmation that the safety feature has been activated. Alternatively, needle retraction can be effected by any other technique for applying the predetermined actuating pressure to the rearwardly protruding head of the actuation member 64.

When the actuating member 64 has moved the latching projections or finger to unlatch or release the latch surface of the head of the needle, the needle is freed for retraction. As the head of the needle is freed, the spring forces or shoots the head of the needle and attached needle into the barrel, and particularly into the chamber 72 in the activating member 64. of course, the device is dimensioned to permit the entire length of the needle to be received into the device so that no portion of the needle protrudes from the front alignment member 24 after retraction.

A vent plug 90 is positioned within the opening of the rear end of actuating member 64 and is adapted to seal the rear of chamber or compartment 72. The vent plug is preferably formed of a resilient porous material that allows air to escape from within the compartment 72 during a blood return. The vent plug 90 is preferably adapted to become clogged when wet so as to prevent any leakage of blood from the rear end of the stylet. In alternative embodiments, the rear end of the compartment 72 may be sealed with a solid sealing member, as long as the compartment 72 is of sufficiently large volume that blood return is not significantly hindered by the back pressure produced therein when the volume is reduced by the influx of blood. The housing 12 and the actuating member 64 are preferably transparent to permit the blood return to be easily visible by the user.

It should be apparent to those skilled in the art that further additions and modifications may be made to the device as disclosed herein. Furthermore, terms and expressions which have been employed are used as terms of description and not of limitation. There is no intention in the use of such terms and expressions of excluding any equivalents of the features shown and described or portions thereof. It is recognized, however, that various modifications are possible within the scope of the invention as claimed.

## Claims

1. An apparatus for intravenous catheter insertion, the apparatus having a housing (12) and a needle (14) extending from the front end (24) of the housing (12) for supporting and guiding a catheter cannula (20) into a blood vessel, a spring (44) within the housing (12) for exerting a force upon the needle (14) to urge the needle into the housing (12), a needle retaining member (36, 36') for releasably holding the needle in its extended position from the housing (12) against the force of the spring (44), and an actuating member (64) which is slidable within the housing (12), has at least a portion protruding from the rear of the housing, has a compartment with an open forward end, and is mounted for movement forward, in response to an applied force, from a first position to a second position, the actuating member, on moving from the first position to the second position, engaging the needle retaining member (36, 36') whereby the needle (14) is forced rearwardly by the spring (44) into the compartment (72),
**characterised in that**
the actuating member (64) and the housing (12) have cooperating locking means (76, 80, 84) operable to lock the actuating member (64) in said first position against force applied to the actuating member up to a predetermined limit, the locking means (76, 80, 84) being operable to release the actuating member upon application of a force to the actuating member (64) in excess of said predetermined limit, thereby allowing displacement of the actuating member (64) to the second position to engage the needle retaining member (36, 36') to effect retraction of the needle (14).

2. An apparatus according to claim 1, in which said locking means (76, 80, 84) have cooperating angled surfaces (80a, 84a), whereby on the application of a force in excess of said predetermined limit to displace the actuating member (64) from said first position to said second position said cooperating angled surfaces provide a radial deforming force as the actuating member moves towards said second position which deforming force is released in a snap as the actuating member moves into said second position.

3. An apparatus according to claim 1 or 2, in which the needle retaining member (36, 36') has an axially elongated radially displaceable arm (46, 46'), wherein upon displacement of the actuating member from the first position to the second position the arm is displaced radially outwardly to effect the retraction of the needle.

4. An apparatus according to claim 1 or 2, in which the needle retaining member (36, 36') has latching projections (46, 46') for engagement with the needle, and wherein the actuating member (64) is moved into engagement with the latching projections (46, 46') on displacement of the actuating member from its first position to its second position to effect the retraction of the needle (14).

5. An apparatus according to claim 4, wherein the latching projections (46') are joined together along the respective sides thereof to form a fluid seal with the needle (14).

6. An apparatus according to any of the preceding claims, further **characterised by** vent means (90) connected with the container (72) for venting gas from within the container while retaining blood therein.

7. An apparatus according to claim 6, wherein the vent means (90) is formed of a porous material for selectively allowing gas to vent from the container (72) while blocking fluid flow.

8. An apparatus according to any of the preceding claims, wherein the needle retaining element (36, 36') is disposed within the housing (12).

9. An apparatus for intravenous catheter insertion, the apparatus having a housing (12) and a needle (14) extending from the front end (24) of the housing (12) for supporting and guiding a catheter cannula (20) into a blood vessel, a spring (44) within the housing (12) for exerting a force upon the needle (14) to urge the needle into the housing (12), a needle retaining member (36, 36') for releasably holding the needle in its extended position from the housing (12) against the force of the spring (44), and an actuating member (64) which is slidable within the housing (12), has at least a portion protruding from the rear of the housing, has a compartment with an open forward end, and is mounted for movement forward, in response to a predetermined force acting on the actuating member, from a first position to a second position, the actuating member, on moving from the first position to the second position, engaging the needle retaining member (36, 36') whereby the needle (14) is forced rearwardly by the spring (44) into the compartment (72),
**characterised in that**
the actuating member (64) and the housing (12) are provided with cooperating radially extending locking means (76, 80, 84) which releasably hold the actuating member (64) in said first position and which have cooperating angled surfaces (80a, 84a), whereby on movement of the actuating member from said first position to said second position said cooperating angled surfaces provide a radial deforming force as the actuating member moves towards said second position which deforming force is released in a snap as the actuating member moves into said second position to engage the needle retaining member to effect retraction of the needle (14).

## Patentansprüche

1. Gerät für die intravenöse Kathetereinführung, wobei das Gerät ein Gehäuse (12) und eine Nadel (14), die sich vom vorderen Ende (24) des Gehäuses (12) zum Stützen und Führen einer Katheterkanüle (20) in ein Blutgefäß erstreckt, eine Feder (44) innerhalb des Gehäuses (12) zum Aufbringen einer Kraft auf die Nadel (14), um die Nadel in das Gehäuse (12) zu drücken, ein Nadelhaltemittel (36, 36') zum lösbaren Halten der Nadel in ihrer ausgefahrenen Stellung aus dem Gehäuse (12) gegen die Kraft der Feder (44), sowie ein Betätigungsmittel (64) aufweist, das im Gehäuse (12) verschiebbar ist, zumindest einen Teil aufweist, der von der Rückseite des Gehäuses vorsteht, ein Fach mit einem offenen Vorderende aufweist und so befestigt ist, dass es als Reaktion auf eine aufgebrachte Kraft von einer ersten Position in eine zweite Position nach vorne bewegt werden kann, wobei das Betätigungselement bei der Bewegung von der ersten Position in die zweite Position in das Nadelhaltemittel (36, 36') eingreift, wodurch die Nadel (14) von der Feder (44) nach hinten in das Fach (72) gedrückt wird, **dadurch gekennzeichnet, dass** das Betätigungsmittel (64) und das Gehäuse (12) zusammenwirkende Arretiermittel (76, 80, 84) aufweisen, die das Betätigungsmittel (64) in der ersten Position gegen die auf das Betätigungsmittel aufgebrachte Kraft bis zu einer vorbestimmten Grenze arretieren können, wobei die Arretiermittel (76, 80, 84) das Betätigungsmittel bei Aufbringung einer Kraft auf das Betätigungsmittel (64), die über die vorbestimmte Grenze hinaus geht, freigeben können, wodurch die Verschiebung des Betätigungsmittels (64) in die zweite Position zum Eingriff mit dem Nadelhaltemittel (36, 36') zum Zurückziehen der Nadel (14) möglich wird.

2. Gerät nach Anspruch 1, worin die Arretiermittel (76, 80, 84) zusammenwirkende abgewinkelte Flächen (80a, 84a) aufweisen, wobei bei Aufbringung einer Kraft, die über die vorbestimmte Grenze hinaus geht, zum Verschieben des Betätigungsmittels (64) aus der ersten Position in die zweite Position die zusammenwirkenden abgewinkelten Flächen eine radiale Verformungskraft ausüben, während sich das Betätigungsmittel in die zweite Position bewegt, wobei diese Verformungskraft sofort wegfällt, wenn sich das Betätigungsmittel in die zweite Position bewegt.

3. Gerät nach Anspruch 1 oder 2, worin das Nadelhaltemittel (36, 36') einen axial verlängerten, radial verschiebbaren Arm (46, 46') aufweist, worin der Arm bei Verschiebung des Betätigungsmittels aus der ersten Position in die zweite Position radial nach außen verschoben wird, um die Nadel zurückzuziehen.

4. Gerät nach Anspruch 1 oder 2, worin das Nadelhaltemittel (36, 36') Sperrvorsprünge (46, 46') zum Eingriff mit der Nadel aufweist und worin das Betätigungsmittel (64) bei Verschiebung des Betätigungsmittels aus seiner ersten Position in die zweite Position zum Zurückziehen der Nadel (14) in Eingriff mit den Sperrvorsprüngen (46, 46') geschoben wird.

5. Gerät nach Anspruch 4, worin die Sperrvorsprünge (46, 46') an ihren Seiten jeweils miteinander verbunden sind, um eine Flüssigkeitsdichtung mit der Nadel (14) zu formen.

6. Gerät nach einem der vorhergehenden Ansprüche, ferner **gekennzeichnet durch** ein Entlüftungsmittel (90), das mit dem Behälter (72) verbunden ist, um Gas aus dem Inneren des Behälters zu entfernen und Blut darin zu halten.

7. Gerät nach Anspruch 6, worin das Entlüftungsmittel (90) aus einem porösen Material besteht, um die selektive Entlüftung des Gases aus dem Behälter (72) unter Blockierung eines Flüssigkeitsflusses zu gestatten.

8. Gerät nach einem der vorhergehenden Ansprüche, worin das Nadelhaltemittel (36, 36') innerhalb des Gehäuses (12) angeordnet ist.

9. Gerät für die intravenöse Kathetereinführung, wobei das Gerät ein Gehäuse (12) und eine Nadel (14), die sich vom vorderen Ende (24) des Gehäuses (12) zum Stützen und Führen einer Katheterkanüle (20) in ein Blutgefäß erstreckt, eine Feder (44) innerhalb des Gehäuses (12) zum Aufbringen einer Kraft auf die Nadel (14), um die Nadel in das Gehäuse (12) zu drücken, ein Nadelhaltemittel (36, 36') zum lösbaren Halten der Nadel in ihrer ausgefahrenen Stellung aus dem Gehäuse (12) gegen die Kraft der Feder (44), sowie ein Betätigungsmittel (64) aufweist, das im Gehäuse (12) verschiebbar ist, zumindest einen Teil aufweist, der von der Rückseite des Gehäuses vorsteht, ein Fach mit einem offenen Vorderende aufweist und so befestigt ist, dass es als Reaktion auf eine aufgebrachte Kraft von einer ersten Position in eine zweite Position nach vorne bewegt werden kann, wobei das Betätigungselement bei der Bewegung von der ersten Position in die zweite Position in das Nadelhaltemittel (36, 36') eingreift, wodurch die Nadel (14) von der Feder (44) nach hinten in das Fach (72) gedrückt wird, **dadurch gekennzeichnet, dass** das Betätigungsmittel (64) und das Gehäuse (12) zusammenwirkende, sich radial erstreckende Arretiermittel (76, 80, 84) aufweisen, die das Betätigungsmittel (64) lösbar in der ersten Position halten und die zusammenwirkende abgewinkelte Flächen (80a, 84a) aufweisen, wobei die zusammenwirkenden abgewinkelten Flächen bei der Bewegung des Betätigungsmittels von der ersten Position in die zweite Position eine radiale Verformungskraft ausüben, während sich das Betätigungsmittel in die zweite Position bewegt, wobei diese Verformungskraft sofort wegfällt, wenn sich das Betätigungsmittel in die zweite Position bewegt, um in das Nadelhaltemittel zum Zurückziehen der Nadel (14) einzugreifen.

## Revendications

1. Dispositif pour l'insertion intraveineuse d'un cathéter, le dispositif comprenant un logement (12) et une aiguille (14) s'étendant à partir de l'extrémité avant (24) du logement (12) pour supporter et guider une canule de cathéter (20) dans un vaisseau sanguin, un ressort (44) à l'intérieur du logement (12) pour exercer une force sur l'aiguille (14) afin de pousser l'aiguille dans le logement (12), un élément de retenue d'aiguille (36, 36') pour maintenir l'aiguille d'une façon libérable dans sa position étendue à partir du logement (12) contre la force du ressort (44), et un élément de commande (64) pouvant coulisser à l'intérieur du logement (12), comprenant au moins une partie qui se projette à partir de l'arrière du logement, comprenant un compartiment présentant une extrémité avant ouverte et monté de manière à effectuer un déplacement vers l'avant en réaction à une force appliquée, entre une première position et une deuxième position, l'élément de commande, lorsqu'il se déplace depuis la première position vers la deuxième position, engageant l'élément de retenu d'aiguille (36, 36') d'une manière telle que l'aiguille (14) soit forcée à reculer par le ressort (44) dans le compartiment (72),
**caractérisé en ce que**:
l'élément de commande (64) et le logement (12) comprennent des moyens de verrouillage coopérants (76, 80, 84) actionnables pour verrouiller l'élément de commande (64) dans ladite première position contre une force appliquée à l'élément de commande jusqu'à une limite prédéterminée, les moyens de verrouillage (76, 80, 84) pouvant être actionnés pour libérer l'élément de commande lors de l'application d'une force à l'élément de commande (64) dépassant ladite limite prédéterminée, permettant ainsi le déplacement de l'élément de commande (64) vers la deuxième position dans le but d'engager l'élément de retenue d'aiguille (36, 36') afin de procéder au retrait de l'aiguille (14).

2. Dispositif selon la revendication 1, dans lequel lesdits moyens de verrouillage (76, 80, 84) présentent des surfaces obliques coopérantes (80a, 84a), dans lequel, lors de l'application d'une force dépassant ladite limite prédéterminée dans le but de déplacer l'élément de commande (64) de ladite première position dans ladite deuxième position, lesdites surfaces obliques coopérantes procurent une force de déformation radiale lorsque l'élément de commande se déplace en direction de ladite deuxième position, ladite force de déformation étant relâchée instantanément lorsque l'élément de commande se déplace dans ladite deuxième position.

3. Dispositif selon la revendication 1 ou 2, dans lequel l'élément de retenue d'aiguille (36, 36') comprend un bras axialement allongé et radialement déplaçable (46, 46'), dans lequel, lors du déplacement de l'élément de commande depuis la première position vers la deuxième position, le bras est déplacé radialement vers l'extérieur afin de procéder au retrait de l'aiguille.

4. Dispositif selon la revendication 1 ou 2, dans lequel l'élément de retenue d'aiguille (36, 36') comprend des saillies de verrouillage (46, 46') à engager avec l'aiguille, et dans lequel l'élément de commande (64) est déplacé en engagement avec les saillies de verrouillage (46, 46') lors du déplacement de l'élément de commande depuis sa première position vers sa deuxième position afin de procéder au retrait de l'aiguille (14).

5. Dispositif selon la revendication 4, dans lequel les saillies de verrouillage (46') sont jointes les unes aux autres le long des côtés respectifs de celles-ci afin de former une étanchéité au fluide avec l'aiguille (14).

6. Dispositif selon l'une quelconque des revendications précédentes, **caractérisé en outre par** un moyen d'évacuation (90) connecté au réservoir (72) pour évacuer le gaz depuis l'intérieur du réservoir tout en retenant le sang à l'intérieur de celui-ci.

7. Dispositif selon la revendication 6, dans lequel le moyen d'évacuation (90) est constitué d'une matière poreuse afin de permettre d'une façon sélective au gaz d'être évacué hors du réservoir (72) tout en bloquant l'écoulement de fluide.

8. Dispositif selon l'une quelconque des revendications précédentes, dans lequel l'élément de retenue d'aiguille (36, 36') est disposé à l'intérieur du logement (12).

9. Dispositif pour l'insertion intraveineuse d'un cathéter, le dispositif comprenant un logement (12) et une aiguille (14) s'étendant à partir de l'extrémité avant (24) du logement (12) pour supporter et guider une canule de cathéter (20) dans un vaisseau sanguin, un ressort (44) à l'intérieur du logement (12) pour exercer une force sur l'aiguille (14) afin de pousser l'aiguille dans le logement (12), un élément de retenue d'aiguille (36, 36') pour maintenir l'aiguille d'une façon libérable dans sa position étendue à partir du logement (12) contre la force du ressort (44), et un élément de commande (64) pouvant coulisser à l'intérieur du logement (12), comprenant au moins une partie qui se projette à partir de l'arrière du logement, comprenant un compartiment présentant une extrémité avant ouverte et monté de manière à effectuer un déplacement vers l'avant en réaction à une force prédéterminée agissant sur l'élément de commande, entre une première position et une deuxième position, l'élément de commande, lorsqu'il se déplace depuis la première position vers la deuxième position, engageant l'élément de retenu d'aiguille (36, 36') d'une manière telle que l'aiguille (14) soit forcée à reculer par le ressort (44) dans le compartiment (72),
**caractérisé en ce que**:
l'élément de commande (64) et le logement (12) sont pourvus de moyens de verrouillage coopérants s'étendant radialement (76, 80, 84) maintenant d'une façon libérable l'élément de commande (64) dans ladite première position et présentant des surfaces obliques coopérantes (80a, 84a), dans lequel, lors du déplacement de l'élément de commande depuis ladite première position vers ladite deuxième position, lesdites surfaces obliques coopérantes procurent une force de déformation radiale lorsque l'élément de commande se déplace en direction de ladite deuxième position, ladite force de déformation étant relâchée instantanément lorsque l'élément de commande se déplace dans ladite deuxième position dans le but d'engager l'élément de retenue d'aiguille afin de procéder au retrait de l'aiguille (14).
